# EUROPÄISCHE PATENTANMELDUNG

(11) **EP 0 647 588 A1**
(43) Veröffentlichungstag der Anmeldung: **12.04.1995**
(21) Anmeldenummer: 94113314.2
(22) Anmeldetag: 25.08.1994
(51) Int. Cl.: C01B 3/12, C07C 29/151, C07C 31/04

(54) **Verfahren und Vorrichtung zur Erzeugung von Methanol**

(30) Priorität: 26.08.1993 DE 4328644
(71) Anmelder: BFI ENTSORGUNGSTECHNOLOGIE GmbH, D-40883 Ratingen (DE)
(72) Erfinder: Münch, Herbert, D-46244 Bottrop/Feldhausen (DE); Herbermann, Michael, D-45966 Gladbeck (DE); Wintrich, Franz, D-45309 Essen (DE); Ruppert, Joachim, D-44795 Bochum (DE)

(57) **Zusammenfassung**

Die vorliegende Erfindung betrifft ein Verfahren zur Erzeugung von Methanol durch Gewinnung von Synthesegas in einem Vergaser, bevorzugt in einem Schlackenbad- oder einem Zyklonvergaser, wobei als Einsatzmaterial in einen Vergaser bevorzugt Abfälle oder Abfälle enthaltende Materialien eingesetzt werden, Umsetzung des Synthesegases mit Wasserdampf in einer Hochtemperatur-Konvertierung, Entfernung der Schwefelverbindungen und von CO₂ bevorzugt in getrennten Reinigungsstufen und Erzeugung von Methanol aus dem gereinigten Synthesegas in einer Methanol-Anlage mit Methanol-Zwischenabzug.

## Beschreibung

Die vorliegende Erfindung betrifft ein Verfahren zur Erzeugung Von Methanol durch Gewinnung von Synthesegas in einem Vergaser, bevorzugt in einem Schlackenbad- oder einem Zyklonvergaser, wobei als Einsatzmaterial in den Vergaser bevorzugt Abfälle oder Abfälle enthaltende Materialien eingesetzt werden, Umsetzung des Synthesegases mit Wasserdampf in einer Hochtemperatur-Konvertierung, Entfernung der Schwefelverbindungen und von CO₂ bevorzugt in getrennten Reinigungsstufen und Erzeugung von Methanol aus dem gereinigten Synthesegas in einer Methanol-Anlage mit Methanol-Zwischenabzug.

Die Methanolerzeugung ist, wie dem Fachmann bekannt ist, eines der technischen Basisverfahren. Methanol ist eine wichtige Grundchemikalie zur Erzeugung von Formaldehyd, der in der Holzindustrie in großen Mengen benötigt wird und von Methyltertiärbuthyläther, der in zunehmenden Maße als sehr wirksame Komponente anstelle von Benzol und Bleiverbindungen Kraftstoffen zugesetzt wird, um die Klopffestigkeit zu verbessern.

Um Methanol wirtschaftlich erzeugen zu können, erfolgt die Produktion in Großanlagen mit Kapazitäten der Größenordnung von 400.000 bis 600.000 t/a.

Das benötigte Synthesegas wird überwiegend aus schweren Rückstandsölen aus der Mineralöl verarbeitenden Industrie in Vergasern wie dem Shell- oder Texaco-Vergaser erzeugt.

Die erforderlichen Mengen an Rückstandsölen zur Auslastung der Methanol-Großanlagen fallen in üblichen Raffinerien vorzugsweise als Destillationsrückstände an.

Bei Einsatz von Kohlenstoff enthaltenden Materialien in Vergaser, die nicht an einem Ort in ausreichender Menge zur Verfügung stehen, wo demgemäß Sammlung und Transport der Einsatzmaterialien im Hinblick auf die Wirtschaftlichkeit der Vergasung und Methanolerzeugung eine erhebliche Bedeutung haben, sind die genannten Großanlagen wenig geeignet. Es besteht daher trotz hochentwickelter Vergasungs- und Methanolproduktionstechnologie die Aufgabenstellung, kleine wirtschaftliche Anlagen zur Verfügung zu stellen, die beispielsweise geeignet sind, Kohlenstoff enthaltende Abfälle bzw. solche Abfälle enthaltende Einsatzgemische z. B. mit Ölen, Biomasse, Kohle und dergleichen zu vergasen.

Der Anmelderin ist es gelungen, diese Aufgabe durch ein Verfahren zu lösen, daß dadurch gekennzeichnet ist, das in einem Vergaser erzeugtes Synthesegas einer Hochtemperatur-Konvertierung zugeführt wird und daß aus dem konvertiertem Gas nach Gasreinigung Methanol in einer Methanol-Anlage mit Methanol-Zwischenabzug erzeugt wird.

Grundsätzlich können die bekannten Vergaser, wie z.B. Shell-Vergaser, Texaco-Vergaser, Wirbelbett-Vergaser, Lurgi-Vergaser, Koppers-Totzek-Vergaser oder Flugstrom-Vergaser eingesetzt werden.

Hierzu ist jedoch festzustellen, daß mit Ausnahme des Vergasers vom Lurgi-Typ und des Wirbelbett-Vergasers z.B. des sogenannten HTW-Vergasers (Hochtemperatur-Winkler-Vergaser) - wobei beide jedoch in einem relativ niedrigen Temperaturbereich von 600 bis 900 °C betrieben werden und damit Asche anstelle der erwünschten, nicht eluierbaren Schlacke erzeugt wird - das Einsatzmaterial einer Feinzerkleinerung unterworfen werden muß, etwa im Bereich von 0,3 bis 0,5 mm.

Die Zerkleinerung ist mit hohem technischen und wirtschaftlichen Aufwand verbunden.

Auch zur Vergasung von Kohlenstoff enthaltenden Abfällen ist die Zerkleinerung derselben eine Voraussetzung, einmal um sie schlammartig oder als Pul ver in den Vergaser eintragen zu können und zum anderen, um eine vollständige Umsetzung zu erzielen.

Umfangreiche Abfallvergasungsversuche wurden von Texaco-Inc. durchgeführt. Die fein zerkleinerten Abfälle wurden im Gemisch mit zerkleinerter Kohle als slurry (Schlamm) in den Vergaser eingeführt. Beispielhaft sei auf eine Veröffentlichung hingewiesen: L. A. Davis, J. E. Miranda, Montebello, Research Laboratory, Texaco Inc., Montebello, California 90640, März 1960, "Texaco hazardous waste gasification".

Auch die Vergasung von getrocknetem Klärschlamm durch Flugstromvergasung ist bekannt. Hierbei wird gemahlener, trockener Klärschlamm mit einem Trägergas in den Vergasungsreaktor eingeführt. Bei der Vergasung von Kunststoffabfällen ist eine Mahlung auf < 0,5 mm erforderlich.

Die Erzeugung von Methanol aus Abfällen verteuert sich durch die aufwendige Zerkleinerung, aber auch durch das Einbringen großer Mengen Trägergas in den Vergasungsreaktor sehr erheblich.

In Wirbelbett-Vergasern können zwar Materialien, die das Wirbelbett bilden, insbesondere Kohle, eine Korngröße bis zu 6 mm aufweisen. Um eine vollständige Vergasung zu erzielen, muß jedoch ein Teil des hinter dem Vergaser anfallenden Staubs in den Reaktor rückgeführt werden.

Erfindungsgemäß bevorzugt sind daher Schlackenbad-Vergaser und Zyklonvergaser. Als Beispiel für einen bereits sehr früh entwickelten Schlackenbad-Vergaser sei der Rummel-Vergaser genannt.

Ein neu entwickelter und erfindungsgemäß bevorzugter Schlackenbad-Vergaser ist jedoch der Thermoselect-Vergaser.

Der Thermoselect-Vergaser enthält einen beheizten Entgasungskanal, in dem verdichtete Einsatzmaterialien unter Luftauschluß entgast werden. Gleichzeitig tritt zunehmend Verkohlung ein. Die Einsatzmaterialien können beliebige Gemische sein und auch anorganische Bestandteile enthalten. Der Vergaser ist insbesondere für die Vergasung praktisch beliebiger Abfallgemische bzw. von Gemischen von Abfällen mit anderen Kohlenstoff enthaltenden Materialien geeignet. Eine Zerkleinerung ist nicht erforderlich. So können beispielsweise Autoreifen nur grob zerkleinert, stückig zugeführt werden, ferner Hausmüll einschließlich der vegetabilischen, Plastik- , Chlor- und andere Komponenten enthaltenden Anteile, ohne das eine aufwendige Sortierung erforderlich ist.

Die entgasten organischen und anorganischen Materialien werden anschließend bei Temperaturen von etwa 1.400 bis 2.000 °C in einem Hochtemperaturreaktor unter dosierter Sauerstoffzugabe vergast bzw. verflüssigt, wobei in der heißesten Zone die Temperatur auch oberhalb 2.000 °C liegen kann.

Auch das im Entgasungskanal entstehende Gas wird dem Hochtemperaturreaktor zugeführt. Die flüssige Schlacke wird einem Homogenisierungsreaktor zugeführt, der bei vergleichbarer Temperatur wie der Vergasungsreaktor arbeitet, in dem aufgrund der unterschiedlichen Dichte eine Trennung in flüssiges Metall und in flüssige Schlacke eintritt. Die Schlacke läßt man zu festem, glasartigem, nicht eluierbarem Material erstarren, der zweite Reaktor wird bevorzugt mit Synthesegas und Sauerstoff beheizt. Das entstehende CO₂ gelangt bevorzugt in den ersten Hochtemperaturreaktor.

Das Thermoselect-Verfahren ist beispielsweise in der Patentschrift P 4 130 416 näher beschrieben.

Ein weiterer, erfindungsgemäß bevorzugter Vergasungsreaktor ist ein Zyklonreaktor, in dem die Einsatzmaterialien, Sauerstoff und Dampf tangential in den Vergasungsreaktor eingeführt werden. Da in dem Reaktor stark turbulente Strömung und hierdurch intensive Mischung eintritt, lassen sich Abfallmaterialien bis zu einer Größe von ca. 15 mm vergasen.

Es ist dem Fachmann bekannt, das auch hier kleinere Korngrößen eingesetzt werden können, der wirtschaftliche Aufwand wird hierdurch jedoch erhöht.

Beispielhaft sei auf den Zyklonvergaser hingewiesen, der in der Europäischen Patentanmeldung Nr. 9 2 11 11 06.8 offenbart ist. In diesem Reaktor tritt eine Strömung, abhängig von Einsatzgeschwindigkeit bzw. Verweilzeit, von der Temperatur und vom Reaktorradius auf, die nicht nur aus einer tangentialen, einer zum Zyklonausgang wirkenden und einer radialen Komponente besteht, sondern in der Strömung tritt zusätzlich ein Drall unter Wirbelausbildung auf, wobei die Drallstärke zu sogenanntem Wirbelaufplatzen der Strömung führt, d. h. es treten Strömungsablösungen bzw. -instabilitäten auf, die zu hohen Turbulenzen im Reaktor führen, wobei der Drall

S = D/I x R

ist.

Hier ist D der Drehimpulstrom, I der Axialimpulsstrom und R der Reaktorradius. In einer solchen Strömung treten für die Vergasung besonders günstige kinetische Verhältnisse auf, so daß eine starke Zerkleinerung des Abfalleinsatzproduktes nicht erforderlich ist.

Auch der Zyklon-Vergaser eignet sich sehr gut zur Vergasung von Abfallgemischen und solche Gemische enthaltende Materialien.

In dem der Vergasung erfindungsgemäß folgenden Schritt erfolgt eine Konvertierung, bevorzugt in Abwesenheit von Katalysatoren, bei der im Temperaturbereich von 600 bis 1.200 °C gearbeitet wird, also bei wesentlich höherer Temperatur, als dies dem Stand der Technik entspricht.

Unter Konvertierungsgleichgewicht versteht man die Gleichgewichtsreaktion zwischen Kohlenmonoxid und Wasserdampf auf der einen Seite und Kohlendioxid und Wasserstoff auf der anderen Seite. Die Reaktion in Richtung Kohlendioxid und Wasserstoff ist schwach exotherm.

CO + H₂O <-> CO₂ + H₂ - 41,0 kJ

Die Gleichgewichtsreaktion wird technisch hauptsächlich zur Erzeugung von Wasserstoff durch Umsetzung von im Synthesegas enthaltenen Kohlenmonoxid mit Wasserdampf genutzt.
Durch die Konvertierungsreaktion kann auch ein für die Weiterverarbeitung von Synthesegas beispielsweise zu Methanol erforderliches CO/H₂-Verhältnis eingestellt werden.

Nach dem Stand der Technik unterscheidet man zwischen einer Hochtemperatur-Konvertierung bei ca. 280 bis 350 °C, bei der man mit einem Eisen-/Chromoxyd-Katalysator arbeitet, der relativ unempfindlich gegen Schwefelwasserstoff im Synthesegas ist und der später entwickelten Tieftemperatur-Konvertierung bei ca. 180 bis 260 °C. Bei dieser Temperatur läßt sich das Konvertierungsgleichgewicht zu H₂ und CO₂ bis auf einen Restgehalt von ca. 0,2 bis 0,4 Volumen-% CO verschieben, im Unterschied zu 4 bis 6 Volumen-% bei der Hochtemperatur-Konvertierung.

Die Tieftemperatur-Konvertierung erfolgt in Gegenwart Kupfer enthaltender Katalysatoren, im allgemeinen CuO/ZnO/Cr₂O₃-Gemische, die sehr empfindlich gegen Schwefelwasserstoff sind.

Die Tieftemperatur-Konvertierung wurde technisch erst möglich, nachdem es gelungen war, Schwefelwasserstoff bis auf Spuren aus dem Synthesegas zu entfernen. Dies gelang durch Überleiten des Synthesegases über ZnO-Betten unter Bildung von ZnS.

Häufig wird in modernen Anlagen die Tieftemperatur-Konvertierung hinter eine Hochtemperatur-Konvertierung geschaltet, wobei in letzterer ein Teil des Schwefelwasserstoffs an den Eisen-/Chromoxyd-Katalysator gebunden wird. Anschließend gelangt das Gas nach Durchströmen von ZnO-Türmen zur Tieftemperatur-Konvertierung.

Da die katalytische Konvertierung technisch und wirtschaftlich aufwendig ist, hat sich die Fachwelt auch darum bemüht, die gewünschte Wasserstoffmenge ohne katalytische Konvertierung zu erzeugen.

Beispielhaft sei die europäische Patentanmeldung Nr. 0 167 101 genannt, gemäß der der Reaktionsraum eines sogenannten Steamreformers zur Umsetzung von Methan mit Wasserdampf, eine Membran aus Palladium aufweist, durch welche bei 500 bis 1000 °C Wasserstoff kontinuierlich abgezogen wird, so daß aus dem erzeugten Synthesegas gemäß

CH₄ + H₂O <-> 3H₂ + CO

das Gleichgewicht fortlaufend zum Wasserstoff verschoben wird. Nach dieser Patentanmeldung läßt sich die eingesetzte Methanmenge durch die Wasserstoffabtrennung von 8,9 auf 1,1 Volumen-% vermindern, wobei jedoch die CO-Menge von 7,5 auf 8,3 Volumen-% geringfügig ansteigt.

Nach wie vor besteht die Aufgabenstellung, das Konvertierungsgleichgewicht zur Umwandlung von Synthesegas in ein Gasgemisch mit höherem Wasserstoffanteil zu verschieben, ohne daß Katalysatoren erforderlich sind. Hierbei ist vor allem von Bedeutung, eine genügend schnelle Gleichgewichtseinstellung herbeizuführen, ohne daß durch Anwendung hoher Temperatur eine Verschiebung zu einem Gasgemisch stattfindet, das einen zu hohen Anteil an CO und Wasserdampf enthält.

Eine Lösung dieser Aufgabenstellung ist der Anmelderin dadurch gelungen, daß erfindungsgemäß die Konvertierung bevorzugt in Abwesenheit eines Katalysators im Temperaturbereich von 600 bis 1200 °C erfolgt, bevorzugt von 600 bis 1000 °C und besonders bevorzugt von 700 bis 900 °C und anschließend eine Abschreckung des Gasgemisches.

Nach dem Stand der Technik wird das rohe Synthesegas üblicherweise zunächst durch indirekten Wärmetausch unter Dampferzeugung und anschließend durch Quenchen mit Wasser auf unter 100 °C abgekühlt. Anschließend wird es einer Gasreinigung zugeführt.

Erst nach erfolgter Reinigung gelangt das Gasgemisch in die Konvertierung.

Hierzu wird im Gegensatz zu diesem Stand der Technik erfindungsgemäß das rohe Synthesegas direkt dem Konvertierungsreaktor zugeführt. Alternativ kann jedoch zwischen Vergaser und Konvertierungsreaktor zur Einstellung der gewünschten Temperatur ein indirekter Wärmetauscher, bevorzugt zur Dampferzeugung, geschaltet sein.

Der Konvertierungsreaktor ist so ausgelegt, daß angepaßt an die Auslegung des Vergasers, die erfindungsgemäßen Verweilzeiten von 0,1 bis 5 Sekunden eingestellt werden können.

Der Konvertierungsreaktor besitzt eine Zone bzw. einen Bereich, in den das rohe Synthesegas eingeführt und konvertiert wird und besitzt wenigstens einen Synthesegaseingang, er kann jedoch auch mehrere Eingänge aufweisen, die an unterschiedlichen Stellen der Konvertierungszone angeordnet sind. Ferner besitzt der Konvertierungsbereich wenigstens eine Wasser- bzw. Dampfzuführung, die so ausgebildet ist, daß das Wasser fein verteilt eingedüst werden kann. Die Wasser- bzw. Dampfzuführungen befinden sich bevorzugt im Bereich der Synthesegaszuführung, können jedoch auch über den Konvertierungsbereich verteilt sein.

Gasgemisch und Dampf bzw. eingedüstes Wasser strömen bevorzugt im Gleichstrom. Dies kann von oben nach unten oder auch von unten nach oben erfolgen, bevorzugt jedoch von oben nach unten.

Der Konvertierungsreaktor besitzt eine zweite Zone, in der das Gasgemisch abgeschreckt werden kann, d. h. so weit abgekühlt werden kann, daß das Konvertierungsgleichgewicht einfriert. Die Abschreckung erfolgt durch Zuführung von Wasser oder auch Niedertemperaturdampf in diese Zone durch eine oder mehrere Zuführungen, wobei das Wasser eingedüst wird.

Es wird erfindungsgemäß auf 50 bis 200 °C, bevorzugt 50 bis 150 °C und bevorzugt auf 70 bis 130 °C abgeschreckt.

Der Konvertierungsreaktor kann sehr einfach konstruiert sein und beispielsweise als einfaches Rohr ausgebildet sein. Er kann jedoch auch andere dem Fachmann bekannte Ausführungen besitzen. Auf die bekannten homogenen Gasphasereaktoren braucht daher nicht näher eingegangen zu werden.

Der Konvertierungsreaktor kann direkt hinter dem Vergaser angeordnet sein. Zwischen Vergaser und Konvertierungsreaktor kann jedoch auch ein indirekter Wärmetauscher angeordnet sein.

Der Konvertierungsreaktor kann indirekte Wärmetauscher im Konvertierungsbereich als auch im Abschreckbereich aufweisen.

Konvertierungsbereich und Abschreckzone können daher erfindungsgemäß durch Einbau indirekter Wärmetauscher zur Dampferzeugung genutzt werden. Im Konvertierungsreaktor erolgt die Einstellung des Konvertierungsgleichgewichts bei einer Temperatur von 600 bis 1200 °C, bevorzugt von 600 bis 1000 °C und besonders bevorzugt von 700 bis 900 °C. Die Einstellung der Temperatur und des Gleichgewichts erfolgen durch Zusatz von Wasser und/oder Dampf im Bereich der Konvertierungszone, in dem auch das rohe Synthesegas zugeführt wird.

Die Temperatureinstellung kann ferner durch indirekten Wärmetausch vor dem Konvertierungsreaktor, wie oben beschrieben, oder im Reaktor durch entsprechende Einbauten erfolgen sowie durch die Kombination von indirektem Wärmetausch und Quenchen.

Die Untersuchungen der Anmelderin haben gezeigt, daß der Temperaturbereich von 700 bis 900 °C besonders geeignet ist, da hier das Konvertierungsgleichgewicht relativ stark zu Wasserstoff und Kohlendioxid verschoben ist und sich ausreichend schnell, auch ohne Gegenwart von Katalysatoren einstellt.

Bei hohen Temperaturen stellt sich zwar das Gleichgewicht schneller ein, es ist jedoch stärker zu Kohlenmonoxid und Wasserdampf verschoben, während sich bei tiefer Temperatur das Konvertierungsgleichgewicht nur relativ langsam einstellt. Die Verweilzeiten zur Einstellung des Konvertierungsgleichgewichts liegen erfindungsgemäß bei 0,1 bis 5 Sekunden, wobei bei hohen Temperaturen die Verweilzeiten kurz und bei niederen Temperaturen lang gewählt werden.

Die Untersuchungen der Anmelderin haben ergeben, daß durch Wasser- bzw. Dampfzusatz in bestimmter Menge in den Vergasungsreaktor oder in den Konvertierungsreaktor oder in beide bei den erfindungsgemäßen Temperaturen eine schnelle Gleichgewichtseinstellung erfolgt, so daß ohne Katalysator problemlos H₂/CO-Verhältnisse > 2 eingestellt werden können.

Anschließend gelangt das Gasgemisch in den Reaktorbereich, in dem es durch Zuführung von Wasser abgeschreckt wird, d. h. so schnell abgekühlt wird, daß das Konvertierungsgleichgewicht einfriert. Die Abschreckung erfolgt auf eine Temperatur von 0 bis 200 °C, bevorzugt von 5 bis 150 °C und besonders bevorzugt von 10 bis 100 °C.

Obgleich die Abschreckung bevorzugt mit Wasser erfolgt, kann auch Niederdruckdampf teilweise mit dem Wasser eingesetzt werden. Auch eine zusätzliche indirekte Kühlung ist wie oben bereits ausgeführt erfindungsgemäß möglich.

Die folgenden Versuche dienen zur neueren Erläuterung der erfindungsgemäßen Konvertierungsstufe.

Bei den Versuchen wurde in den Vergaser ein Kohlenstoff enthaltendes Material eingesetzt, dessen Zusammensetzung in Tabelle 1 dargestellt ist.

**Tabelle 1**

| | |
|---|---|
| C | 57,8 |
| H | 7,2 |
| N | 7,5 |
| O | 19,5 |
| Asche | 8,0 |

Das Material wurde vor Einsatz in den Vergaser mit Wasser gemischt, so daß die prozentuale Wassermenge bezogen auf das Gesamtgewicht von Trockensubstanz + Wasser bei 3, 30 und 50 Gew.-% lag, entsprechend einer Gesamtmenge von Wasser pro 100 kg Trockensubstanz von 3,1 kg, 39 kg und 100 kg. Pro 100 kg Kohlenstoff enthaltendem Material wurden 95,7 kg Sauerstoff (+ 0,94 kg Stickstoff) eingesetzt.

Das rohe Gasgemisch gelangte mit 1350 °C in den Konvertierungsreaktor. Bei einem Druck von 1,5 bar wurde die Temperatur des Gasgemischs auf 900 °C, 800 °C und 700 °C durch indirekte Kühlung gesenkt.

Hierbei wurden die in den Tabellen 2 bis 4 wiedergegebenen Ergebnisse erhalten, die sich auf 100 kg Einsatz an Kohlenstoff enthaltendem Material beziehen.

**Tabelle 2**

| Abkühlung auf 900 °C | | | |
|---|---|---|---|
| | 3 Gew.-% H₂O* | 30 Gew.-% H₂O | 50 Gew.-% H₂O |
| Gasmenge in m³ | 577 | 721 | 927 |
| Gasmenge in Nm³ | 199 | 248 | 319 |
| H₂ Vol.-% | 28,2 | 28,3 | 26,1 |
| CO Vol.-% | 39,2 | 25,7 | 15,9 |
| CO₂ Vol.-% | 15,0 | 17,7 | 17,8 |
| H₂O Vol.-% | 14,0 | 25,4 | 37,9 |
| N₂ Vol.-% | 3,6 | 2,9 | 2,3 |
| Wärme MJ | - 167 | - 220 | - 291 |

| | | | |
|---|---|---|---|
| * Die Gewichtsprozente H₂O beziehen sich auf 100 kg Einsatz an C-haltigem Material + Wassermenge. | | | |

**Tabelle 3**

| Abkühlung auf 800 °C | | | |
|---|---|---|---|
| | 3 Gew.-% H₂O* | 30 Gew.-% H₂O | 50 Gew.-% H₂O |
| Gasmenge in m³ | 528 | 660 | 848 |
| Gasmenge in Nm³ | 199 | 248 | 319 |
| H₂ Vol.-% | 29,8 | 30,2 | 27,8 |
| CO Vol.-% | 37,6 | 23,8 | 14,2 |
| CO₂ Vol.-% | 16,6 | 19,6 | 19,6 |
| H₂O Vol.-% | 12,4 | 23,5 | 36,2 |
| N₂ Vol.-% | 3,6 | 2,9 | 2,3 |
| Wärme MJ | - 205 | - 271 | - 357 |

| | | | |
|---|---|---|---|
| * Die Gewichtsprozente H₂O beziehen sich auf 100 kg Einsatz an C-haltigem Material + Wassermenge | | | |

**Tabelle 4**

| Abkühlung auf 700 °C | | | |
|---|---|---|---|
| | 3 Gew.-% H₂O* | 30 Gew.-% H₂O | 50 Gew.-% H₂O |
| Gasmenge in m³ | 478 | 598 | 769 |
| Gasmenge in Nm³ | 199 | 248 | 319 |
| H₂ Vol.-% | 31,7 | 32,6 | 29,9 |
| CO Vol.-% | 35,5 | 21,4 | 12,1 |
| CO₂ Vol.-% | 18,6 | 22,0 | 21,7 |
| H₂O Vol.-% | 10,5 | 21,2 | 34,1 |
| N₂ Vol.-% | 3,6 | 2,9 | 2,3 |
| Wärme MJ | - 246 | - 323 | - 424 |

| | | | |
|---|---|---|---|
| * Die Gewichtsprozente H₂O beziehen sich auf 100 kg Einsatz an C-haltigem Material + Wassermenge | | | |

Die Tabellen zeigen, daß mit zunehmendem Wasseranteil im Einsatzprodukt, entsprechend der zunehmenden exothermen Umsetzung bei Senkung der Temperatur von 900 °C auf 700 °C die Wasserstoffmenge zunimmt und die CO-Menge und H₂O-Menge abnehmen (Wärmetönung bei 900 °C und 50 Gew.-% H₂O: - 291 MJ; Wärmetönung bei 700 °C und 50 Gew.-% H₂O: - 424 MJ).

In den Tabellen 5 bis 7 sind Ergebnisse wiedergegeben, die durch zumindest teilweises Quenchen mit Wasser bzw. mit Wasserdampf erhalten wurden.

Zusätzlich zu den im Einsatzprodukt in dem Vergaser vorhandenen Wasser wurden durch Quenchen weitere 54,05 kg H₂O bzw. im Falle von 3 Gew.-% H₂O im Vergasereinsatzprodukt 144 kg H₂O zugegeben.

**Tabelle 5**

| Abkühlung auf 900 °C | | | | |
|---|---|---|---|---|
| zugesetzte H₂O-Menge in die Vergasung | 3 Gew.-% | 3 Gew.-% | 30 Gew.-% | 50 Gew.-% |
| zugesetzte H₂O-Menge/kg | 54,05 | 144 | 54,05 | 54,05 |
| Gasmenge in m³ | 772 | 1097 | 916 | 1122 |
| Gasmenge in Nm³ | 266 | 378 | 316 | 387 |
| H₂ Vol.-% | 27,9 | 23,9 | 26,2 | 23,6 |
| CO Vol.-% | 22,5 | 11,5 | 16,3 | 11,1 |
| CO₂ Vol.-% | 18,0 | 17,0 | 17,9 | 16,8 |
| H₂O Vol.-% | 28,9 | 45,7 | 37,4 | 46,6 |
| N₂ Vol.-% | 2,7 | 1,9 | 2,3 | 1,9 |
| Wärme MJ (1) | 38 | | - 6 | - 71 |
| Wärme MJ (2) | - 112 | | | |
| Wärme MJ (3) | | - 27 | | |

| | | | | |
|---|---|---|---|---|
| (1) Zugabe von flüssigem H₂O von 25 °C | | | | |
| (2) Zugabe von gasförmigem H₂O von 200 °C | | | | |
| (3) Zugabe von gasförmigem H₂O von 300 °C | | | | |

**Tabelle 6**

| Abkühlung auf 800 °C | | | | |
|---|---|---|---|---|
| zugesetzte H₂O-Menge in die Vergasung | 3 Gew.-% | 3 Gew.-% | 30 Gew.-% | 50 Gew.-% |
| zugesetzte H₂O-Menge/kg | 54,05 | 144 | 54,05 | 54,05 |
| Gasmenge in m³ | 706 | 1004 | 838 | 1026 |
| Gasmenge in Nm³ | 266 | 378 | 316 | 387 |
| H₂ Vol.-% | 29,8 | 25,4 | 28,0 | 25,1 |
| CO Vol.-% | 20,6 | 10,0 | 14,5 | 9,6 |
| CO₂ Vol.-% | 19,9 | 18,5 | 19,6 | 18,3 |
| H₂O Vol.-% | 27,0 | 44,1 | 35,6 | 45,1 |
| N₂ Vol.-% | 2,7 | 1,9 | 2,3 | 1,9 |
| Wärme MJ (1) | - 16 | | - 72 | - 151 |
| Wärme MJ (2) | - 166 | | | |
| Wärme MJ (3) | | 105 | | |

| | | | | |
|---|---|---|---|---|
| (1) Zugabe von flüssigem H₂O von 25 °C | | | | |
| (2) Zugabe von gasförmigem H₂O von 200 °C | | | | |
| (3) Zugabe von gasförmigem H₂O von 300 °C | | | | |

**Tabelle 7**

| Abkühlung auf 700 °C | | | | |
|---|---|---|---|---|
| zugesetzte H₂O-Menge in die Vergasung | 3 Gew.-% | 3 Gew.-% | 30 Gew.-% | 50 Gew.-% |
| zugesetzte H₂O-Menge/kg | 54,05 | 144 | 54,05 | 54,05 |
| Gasmenge in m³ | 641 | 910 | 760 | 931 |
| Gasmenge in Nm³ | 266 | 378 | 316 | 387 |
| H₂ Vol.-% | 32,1 | 27,2 | 30,1 | 26,8 |
| CO Vol.-% | 18,3 | 8,2 | 12,4 | 7,8 |
| CO₂ Vol.-% | 22,2 | 20,3 | 21,8 | 20,0 |
| H₂O Vol.-% | 24,7 | 42,4 | 33,5 | 43,4 |
| N₂ Vol.-% | 2,7 | 1,9 | 2,3 | 1,9 |
| Wärme MJ (1) | - 72 | | - 138 | - 230 |
| Wärme MJ (2) | - 222 | | | |
| Wärme MJ (3) | | - 183 | | |

| | | | | |
|---|---|---|---|---|
| (1) Zugabe von flüssigem H₂O von 25 °C | | | | |
| (2) Zugabe von gasförmigem H₂O von 200 °C | | | | |
| (3) Zugabe von gasförmigem H₂O von 300 °C | | | | |

Die Tabellen 5 bis 7 zeigen, daß durch weitere Zugabe von Wasser in Mengen von 54,04 kg bzw. 144 kg pro kg Kohlenstoff enthaltendem Einsatzmaterial in den Vergaser bei Senkung der Temperatur von 900 °C auf 700 °C durch Quenchen das Verhältnis von erzeugtem Wasserstoff zu Kohlenmonoxid im Konvertierungsgas weiter deutlich gesteigert werden kann.

Das Konvertierungsgasgemisch wurde nach Einstellung des Gleichgewichts bei einer Verweilzeit von 0,1 bis 5 Sekunden durch Eindüsen von Wasser abgeschreckt, so daß das Gleichgewicht einfror.

Tabelle 8 gibt wieder, welches Verhältnis von Wasserstoff zu Kohlenmonoxid sich nach Entfernen von CO₂ und H₂O bei 25 °C eingestellt hat. Tabelle 8 entspricht den Tabellen 2 bis 4.

**Tabelle 8**

| | | | |
|---|---|---|---|
| zugesetzte H₂O-Menge in die Vergasung | 3 Gew.-% | 30 Gew.-% | 50 Gew.-% |
| Gasmenge in Nm³ | 143 | 144 | 145 |

| Konvertierung bei 900 °C | | | |
|---|---|---|---|
| H₂ Vol.-% | 39, 2* | 48,9* | 57,6 |
| CO Vol.-% | 54,5 | 44,4 | 35,1 |
| N₂ Vol.-% | 5,0 | 5,0 | 5,1 |
| H₂/CO | 0,69 | 1,03 | 1,5 |

| Konvertierung bei 800 °C | | | |
|---|---|---|---|
| H₂ Vol.-% | 41,4 | 52,1 | 61,4 |
| CO Vol.-% | 52,5 | 41,1 | 31,4 |
| N₂ Vol.-% | 5,1 | 5,0 | 5,1 |
| H₂/CO | 0,75 | 1,18 | 1,77 |

| Konvertierung bei 700 °C | | | |
|---|---|---|---|
| H₂ Vol.-% | 44,0 | 56,2 | 65,9 |
| CO Vol.-% | 49,3 | 36,9 | 26,7 |
| N₂ Vol.-% | 5,0 | 5,0 | 5,1 |
| H₂/CO | 0,84 | 1,4 | 2,19 |

| | | | |
|---|---|---|---|
| * Differenz zu 100 % jeweils Restmengen an CO₂ und H₂O | | | |

In Tabelle 9 ist die Gleichgewichtseinstellung bei den zusätzlichen, durch Quenchen zugegebenen H₂O-Mengen dargestellt.

Tabelle 9 entspricht den Tabellen 5 bis 7.

**Tabelle 9**

| | | | | |
|---|---|---|---|---|
| zugesetzte Quenchwassermenge/kg | 54,05 | 144 | 54,05 | 54,05 |
| zugesetzte H₂O-Menge in die Vergasung | 3 Gew.-% | 3 Gew.-% | 30 Gew.-% | 50 Gew.-% |
| Gasmenge in Nm³ | 266 | 378 | 316 | 387 |

| Konvertierung bei 900 °C | | | | |
|---|---|---|---|---|
| H₂ Vol.-% | 51,6 | 62,5 | 57,3 | 62,9 |
| CO Vol.-% | 41,7 | 30,1 | 35,7 | 29,6 |
| N₂ Vol.-% | 5,0 | 5,0 | 5,0 | 5,1 |
| H₂/CO | 1,15 | 1,87 | 1,47 | 1,91 |

| Konvertierung bei 800 °C | | | | |
|---|---|---|---|---|
| H₂ Vol.-% | 55,0 | 66,1 | 61,0 | 66,8 |
| CO Vol.-% | 38,0 | 26,0 | 31,6 | 25,5 |
| N₂ Vol.-% | 5,0 | 4,9 | 5,0 | 5,1 |
| H₂/CO | 1,33 | 2,24 | 1,75 | 2,3 |

| Konvertierung bei 700 °C | | | | |
|---|---|---|---|---|
| H₂ Vol.-% | 59,1 | 70,8 | 65,6 | 71,1 |
| CO Vol.-% | 33,7 | 21,4 | 27,0 | 20,7 |
| N₂ Vol.-% | 5,0 | 4,9 | 5,0 | 5,0 |
| H₂/CO | 1,6 | 2,84 | 2,15 | 2,93 |

Die Ergebnisse zeigen, daß durch Zusatz von 144 kg H₂O als Quenchwasser + ca. 3 kg H₂O im Einsatzprodukt bei 700 °C ein Verhältnis von H₂/CO von 2,84 und bei Zusatz von 54,05 kg H₂O als Quenchwasser zu 100 kg H₂O im Einsatzprodukt sogar ein Verhältnis von H₂/CO von 2,93 bei 700 °C erhalten werden kann.

Das erfindungsgemäße Verfahren bietet weitere wichtige Vorteile, so ist dadurch, daß das durch Vergasung erzeugte Gasgemisch nicht vor der Konvertierung abgekühlt, gereinigt und wieder aufgeheizt werden muß, eine erhebliche Energieeinsparung möglich. Der Konvertierungsreaktor kann sehr einfach konstruiert sein, z. B. nur als Rohr mit Wasserdüsen ausgebildet sein. Der vergleichsweise hohe Wasseranteil führt zu einer besseren Feinstaubabscheidung sowie zur besseren Entfernung von HCl, HF, H₂S, NH₃ oder Metalldämpfen, da sich mit Hilfe der Staubkörnchen als Kristallisationskeime leicht H₂O-Tröpfchen bilden, die eine Waschwirkung entfalten.

Das konvertierte Gasgemisch kann einer konventionellen Gasreinigung zugeführt werden.

Bei der üblichen Reinigung von Synthesegas zur Entfernung von Schwefelwasserstoff und CO₂, aber auch von COS, CS₂ und HCN erfolgt eine Wäsche mit speziellen Lösungsmitteln, die weiter unten genannt werden. Mit Hilfe dieser Lösungsmittel werden Schwefelwasserstoff, Kohlensäure sowie COS, HCN und CS₂ gemeinsam entfernt, wobei mit erheblichem technischen Aufwand die absorbierten Gase zumindest weitgehend getrennt aus den Lösungsmitteln wieder abgetrennt werden. Ein sehr verbreitetes Verfahren ist die Rectisol-Wäsche, nämlich die Wäsche des Gases mit tiefgekühltem Methanol.

Die geschilderten naß-physikalischen Wäschen zur Entfernung von Schwefelwasserstoff und Kohlendioxyd COS, CS₂ und HCN und die selektive Gewinnung dieser Gase aus der Waschflüssigkeit sind mit hohen Kosten verbunden. Dies betrifft nicht nur die Investitionskosten sondern auch die Betriebskosten.

Erfindungsgemäß bevorzugt wird daher der als H₂S vorliegende Schwefelgehalt des Gases katalytisch bis zu ≦ 3000 ppm, bevorzugt bis zu ≦ 1500 ppm und besonders bevorzugt bis zu ≦ 1000 ppm entfernt - hier kann beispielsweise das "Clinsulf"-Verfahren eingesetzt werden-. Der als COS und CS₂ vorliegende Schwefelgehalt, soweit dieser in der katalytischen Stufe nicht entfernt wurde, wird durch Hydrolyse an Aluminiumoxid-Katalysatoren und anschließende Adsorption des gebildeten H₂S an Aktivkohle und/oder Adsorption und Umsetzung an dotierter Aktivkohle entfernt. Bei Gas mit einem als H₂S vorliegenden Schwefelgehalt ≦ 3000 ppm, bevorzugt ≦ 1500 ppm wird dieser über dotierter Aktivkohle bis zu einem Schwefelgehalt von < 10 mg/m³ gesenkt. Nachgeschaltet kann zur zusätzlichen Entfernung von H₂S ein Zinkoxidbett sein. Nach der Schwefelreinigung kann Kohlendioxid durch Gaswäsche und/oder Druckwechseladsorption bis auf einen Restgehalt von < 10 Gew.-%, bevorzugt > 0 bis < 5 Gew.-% abgetrennt werden.
Es ist im erfindungsgemäßen Verfahren von Vorteil, die katalytischen Entschwefelungen bei einem Druck von 15 bis 25 bar, bevorzugt 12 bis 18 bar durchzuführen.
Zur Gaswäsche können auch hier die oben genannten Waschlösungen eingesetzt werden.

Erfindungsgemäß werden daher bevorzugt die im Gas vorhandenen Verunreinigungen Schwefelwasserstoff und CO₂ getrennt entfernt. Zusätzlich kann die hydrolytische Umwandlung von COS und CS₂ in H₂S und CO₂ durchgeführt werden. Hierdurch ist die aufwendige Waschstufe, in der die Verunreinigungen gemeinsam entfernt werden und anschließend selektiv aus dem Lösungsmittel abgetrennt werden müssen, nicht mehr erforderlich.

In Abhängigkeit von dem Schwefelwasserstoff-Gehalt kann, wie dargelegt, die erfindungsgemäße Entschwefelung ein- oder zweistufig erfolgen. Bei hohen Schwefelwasserstoff-Gehalten von mehr als 3000 ppm wird das zu reinigende Gas durch eine Katalysatorzone geleitet, in der ein Teil des vorliegenden Schwefelwasserstoffes katalytisch zu SO₂ oxidiert wird. Hierbei wird der Sauerstoff in einer solchen Menge zugegeben, daß man ein Verhältnis von H₂S und SO₂ von etwa 2 : 1 erhält. In Gegenwart eines Katalysators wird nunmehr durch Umsetzung der beiden Gase elementarer Schwefel erzeugt. Bevorzugt wird ein Verhälfnis H₂S zu SO₂ eingestellt, in dem der Schwefelwasserstoff im Überschuß vorliegt. In dieser Stufe wird die Schwefelwasserstoffkonzentration bis zu ≦ 3000 ppm, bevorzugt bis zu ≦ 1500 ppm und besonders bevorzugt bis zu ≦ 1000 ppm gesenkt. Die Katalysatoren für die Schwefelerzeugung sind dem Fachmann bekannt, sie brauchen daher hier nicht näher erläutert werden.

Gase, die einen geringeren als H₂S vorliegenden Schwefelgehalt, als ≦ 3000 ppm, bevorzugt ≦ 1500 ppm und besonders bevorzugt ≦ 1000 ppm enthalten, werden einer zweiten Entschwefelungsstufe zugeführt, in der der Schwefelgehalt in Gegenwart dotierter Aktivkohle bis zu < 10 mg/m³ reduziert wird. Als Dotierung werden Jod und/oder Jodverbindungen eingesetzt. Bevorzugt wird mit Kaliumjodid dotiert.
Weitere erfindungsgemäß eingesetzte Dotierungen sind Eisenverbindungen, insbesondere Eisenkomplex-Verbindungen und Oxidationskatalysatoren.

In dieser Reinigungsstufe werden die restlichen Schwefelverbindungen katalytisch zu Schwefel umgesetzt. Der ggf. erforderliche Sauerstoff wird zudosiert. Der Schwefel wird an der Aktivkohle adsorbiert. Die Reaktionstemperatur liegt im allgemeinen bei 50 bis 100 °C.

Im Synthesegas vorhandenes COS und CS₂ können durch Hydrolyse an Aluminiumoxid-Katalysatoren hydrolytisch bei wenigstens 160 °C in H₂S und CO₂ gemäß folgenden Gleichungen umgewandelt werden:

COS + H₂O <=> H₂S + CO₂

CS₂ + 2 H₂O <=> 2 H₂S + CO₂

Die Hydrolyse kann nach der 1. katalytischen Umsetzungsstufe durchgeführt werden, jedoch grundsätzlich auch vor dieser oder nach der 2. katalytischen Umsetzungsstufe.

Der Schwefelwasserstoff kann anschließend an Aktivkohle adsorbiert werden oder an dotierter Aktivkohle zusätzlich zu elementarem Schwefel umgesetzt werden.

Falls nach der 2. katalytischen Umsetzungsstufe ein Zinkoxidbett vorhanden ist, kann ggf. die COS und CS₂-Hydrolyse entfallen.

Nunmehr kann das im Gas befindliche CO₂ durch Gaswäsche und/oder Druckwechseladsorption bis auf einen Restgehalt von < 10 Gew.-%, bevorzugt > 0 bis < 5 Gew.-% entfernt werden.
Da die Druckwechseladsorption eine dem Fachmann vertraute Technologie ist, soll sie hier nicht näher erläutert werden. Im allgemeinen wird sie bei 5 bis 200 bar, bevorzugt bei 10 bis 100 bar betrieben.

Bei Einsatz konventioneller Gaswäschen können die bekannten Verfahren zur Entfernung von CO₂ bzw. H₂S + CO₂ angewendet werden. Als wichtige technische Gaswäschen seien genannt:
- Rectisol-Verfahren; Methanol als Waschmittel, Temperatur ca. - 75 °C
- Selexol-Verfahren; mit DMEG (Polyethylenglykol-Dimethyläther), Vorteil ist der niedrige Dampfdruck des DMEG
- Purisol-Verfahren; mit NMP (N-Methylpyrolidon), Dampfdruck 400 mal höher als der des DMEG
- Heißpottasche Wäsche; mit Kaliumcarbonat
- Alkazid-Verfahren; mit monomethylsaurem Kalium als Waschmittel (Alkazid-M-Verfahren)
- Amin-Wäschen; mit MEA, DEA und TEA (Mono-, Di- oder Triethanolamin)
- Natronlauge-Wäsche; einziges Verfahren ohne nachgeschaltete Regeneration
- Sulfinol-Verfahren; Absorbens ist eine Mischung aus DIPA, Tetrahydrothiophendioxid und Wasser
- Selexol-A-Verfahren; Mischung aus DIPA und DMPEG
- Amisol-Verfahren; Mischung aus MEA oder DEA mit Methanol und Wasser
- Stretford-Verfahren; Waschlösung aus Natriumcarbonat, Anthrachinondisulfonsäure, Citrat und Natriumvanadat
- Giammarco-Vetrocoke-Verfahren
- Locatverfahren; Absorbens ist eine Eisensalzlösung
- Propylencarbonat-Wäsche
Die erfindungsgemäße getrennte Entfernung von H₂S und CO₂ bietet wesentliche Vorteile gegenüber dem Stand der Technik. Sie ist besonders geeignet für kleine Gasmengen zwischen 5000 und 50000 Nm³/h. Ein up- oder down-scaling ist innerhalb dieses Bereiches unproblematisch.

Die erfindungsgemäßen Reinigungsstufen können ohne Kreislaufführung eines Gasstromes erfolgen. Hierdurch wird insbesondere die Wirtschaftlichkeit erhöht.

In beiden Reinigungsstufen zur Entfernung von H₂S wird elementarer Schwefel gewonnen.

Die erfindungsgemäße Reinigungstechnologie ist sehr flexibel gegenüber schwankenden Gaszusammensetzungen.

Die hohen Investitions- und Betriebskosten der gemeinsamen Wäsche von H₂O + CO₂ und deren getrennt Rückgewinnung entfallen.

Erfindungsgemäß erfolgt die Umsetzung des gereinigten Synthesegases in einem Methanolreaktorsystem, aus dem Methanol in Zwischenabzügen aus dem Reaktionsgleichgewicht entfernt wird. Bei dem Verfahren kann man auf die Kreislauffahrweise verzichten und Kompressionskosten, Katalysatorkosten sowie erhebliche Investkosten einsparen unter Verzicht auf das Kreislaufsystem und durch Verminderung des Reaktorvolumens.

Hierdurch ergeben sich wesentliche wirtschaftliche Vorteile gegenüber dem Stand der Technik.

Dies ist insbesondere von sehr großer Bedeutung, wenn Methanol als Kraftstoff alternativ zu Benzin und Dieselöl eingesetzt werden soll. Da Methanol zu 50 Gew.-% aus Sauerstoff besteht, müssen die Erzeugungskosten des Methanols, trotz höherem Wirkungsgrad als Kraftstoff, erheblich unter den Erzeugungskosten von Benzin und Dieselöl liegen ( siehe z. B. "Alternative Energien für den Straßenverkehr, Methanol", Herausgeber: Der Bundesminister für Forschung und Technologie, Verlag: TÜV-Rheinland GmbH, Köln, 1983).

Im folgenden soll der wichtigste Stand der Technik der Methanolerzeugung kurz geschildert werden.

Durch die insbesondere von ICI entwickelte Feinstreinigung von Synthesegas über Zinkoxydbetten lassen sich bei den modernen Methanolsynthese-Verfahren hochaktive, Kupfer enthaltende Katalysatoren einsetzen. Man kann daher bei niedrigeren Temperaturen arbeiten als bei den Hochdrucksynthesen, wobei die niedrigeren Temperaturen die Gleichgewichtseinstellung mit höherer Methanolkonzentration im Gleichgewicht begünstigen. Hierdurch kann man auf die in den früheren Methanolhochdrucksynthese-Anlagen angewendeten Drücke von ca. 300 bis 350 bar verzichten und bereits bei 60 bis 100 bar Methanol technisch erzeugen. Noch immer ist jedoch die Rückführung einer großen aus CO und H₂ bestehenden Kreislaufgasmenge erforderlich, da das aus dem Reaktor austretende Gleichgewichtsgemisch mit Methanol zunächst abgekühlt wird, das Methanol durch Kondensation abgetrennt wird und das nunmehr nicht mehr zur Gleichgewichtseinstellung befähigte Synthesegas, zunächst durch Wärmetausch erneut auf Reaktionstemperatur aufgeheizt werden muß und dann erneut über den Katalysator geleitet werden muß.

Im Falle des LURGI-Methanolverfahrens wird nur ein Volumenteil des Synthesegases zu Methanol im geraden Durchgang durch den Reaktor umgesetzt, während 4 bis 6 Volumenteile im Kreislauf gefahren werden.

Obgleich sich die Kompressionskosten gegenüber der früheren Hochdruckfahrweise deutlich vermindert haben, ist der Kompressionskostenanteil noch immer ein sehr wichtiger Kostenfaktor. Ferner erfordert das Kreislaufsystem hohe Investitionskosten.

Diesen Verfahren gegenüber wird erfindungsgemäß bei der katalytischen Umsetzung des Synthesegases erzeugtes Methanol aus dem Gleichgewicht entfernt. Dies kann auf unterschiedliche Weise geschehen. Beispielhaft sei die Methanolsynthese in einem Rieselreaktor genannt, in dem ein pulverförmiges Adsorptionsmittel zwischen den Katalysatorpartikeln von oben nach unten durch den Reaktor fließt und hierbei das gebildete Methanol adsorbiert (siehe z. B. K. R. Westerterp, M. Kuczynski, Hydrocarbon Processing, 1986, Nr. 11, Seiten 80 bis 83).

Erfindungsgemäß bevorzugt ist ein Verfahren, bei dem das erzeugte Methanol mittels einer Flüssigkeit aus dem Gleichgewicht extrahiert wird (siehe z. B. K. B. Westerterp, M. Kuczynski, T. M. Bodewes, M. S. A. Vrijland, CHEM.-ING.-TECH. 61, 1989, 3, Seiten 193 - 199).

Bei einem solchen Verfahren wird das Methanol in mehreren Reaktorstufen erzeugt. Hinter jeder Stufe befindet sich ein Absorber, in dem die Extraktion des Methanols erfolgt. Geeignete Extraktionsmittel sind Polyethylenglykoläther, wobei ein besonders geeignetes Extraktionsmittel Tetraethylenglykoldimethyläther ist, jedoch auch andere Extraktionsmittel sind erfindungsgemäß einsetzbar. Durch stufenweise Entspannung kann zunächst gelöstes Synthesegas und anschließend das Methanol gasförmig abgetrennt werden Bei einem 4-stufigen Verfahren entfällt der Synthesegaskreislauf und damit der Kreislauf-Kompressor. Etwa 94 % des eingesetzten Kohlenmonoxyds werden im geraden Durchgang umgesetzt. 4-Stufigkeit ist jedoch nicht erforderlich. Erfindungsgemäß kann auch mit 1 bis mehr als 4 Stufen, z. B. 3 - 5 Stufen gearbeitet werden, wobei sich jedoch Synthesegasumsetzung und Wirtschaftlichkeit verändern. Die Reaktoren sind im allgemeinen Röhrenreaktoren, es können jedoch auch andere Reaktoren, z. B. sogenannte Abschnittsreaktoren, mit Kaltgaskühlung eingesetzt werden. Kühlwasser und Katalysatorverbrauch sind wesentlich geringer als in einem klassischen Niederdruckverfahren. Das im geraden Durchgang nicht umgesetzte Gas kann dem Vergasungsreaktor, im Falle des Thermoselect-Verfahrens, z. B. dem Vergasungs- oder Homogenisierungsreaktor zugeführt werden.

Hierdurch wird eine anderweitige Entsorgung bzw. Verbrennung des Gases vermieden und durch Umsetzung des rückgeführten Kohlenmonoxids wird zusätzlich Wasserstoff erzeugt.

Da das gemäß vorliegender Erfindung eingesetzte Methanolverfahren auch in Gegenwart relativ großer Mengen Kohlenmonoxids eine praktisch vollständige Umsetzung des Wasserstoffs zu Methanol erlaubt, kann ggf. auch ohne Konvertierung gearbeitet werden. Überschüssiges CO am Ausgang der Methanolanlage kann entweder verstromt oder in das Vergasungsverfahren rückgeführt werden. Die Fahrweise ohne Konvertierung führt zu Kosteneinsparungen.

Die erfindungsgemäße Vorrichtung kann im Vergaser- und Hochtemperatur-Konvertierungsbereich mit Brauchwasser aus der Anlage versorgt werden, so daß Frischwasser eingespart werden kann.

Die vorliegende Erfindung betrifft auch eine Vorrichtung zur Durchführung des erfindungsgemäßen Verfahrens, dadurch gekennzeichnet, daß diese wenigstens einen Vergaser aufweist, wenigstens ein Hochtemperatur-Konvertierung, eine Gasreinigung und eine Anlage zur Methanolerzeugung mit Methanol-Zwischenabzug.

Bevorzugt ist der in der Vorrichtung installierte Vergaser ein Schlackenbad-Vergaser und besonders bevorzugt ein Thermoselect-Vergaser.

Einzelheiten zum Thermoselect-Vergaser wurden bereits in der Verfahrensbeschreibung angegeben.

Ein weiterer erfindungsgemäß bevorzugter Vergaser ist ein Zyklon-Vergaser. Auch dieser ist bereits in der Verfahrensbeschreibung erläutert worden.

Der Hochtemperatur-Konvertierungsreaktor weist eine Konvertierungszone und eine Abkühlzone bzw. Abschreckzone mit wenigstens je einer Dampf- bzw. Wasserzuführung auf.

Das Wasser wird bevorzugt in die Abkühlzone eingedüst.

Der Konvertierungsreaktor kann direkt hinter den Vergaser geschaltet sein und über einen Eingang für rohes Synthesegas versorgt werden. Zwischen Vergaser und Hochtemperatur-Konverter kann sich jedoch auch wenigstens ein indirekter Wärmetauscher befinden, um die gewünschte Temperatur im Hochtemperatur-Konvertierungsreaktor einstellen zu können.

Der Hochtemperatur-Konvertierungsreaktor selbst kann sowohl in der Konvertierungszone als auch in der Abkühlungszone indirekte Wärmetauscher aufweisen.

Mit Hilfe der indirekten Wärmetauscher kann Dampf erzeugt werden.

Wasser bzw. Dampf kann erforderlichenfalls auch dem Vergaser zugeführt werden anstelle dem Hochtemperatur-Konvertierungsreaktor.

Erfindungsgemäß bevorzugt ist der Konvertierungsreaktor rohrartig ausgebildet. Er kann jedoch, wie dein Fachmann bekannt ist auch andere Konstruktionen besitzen.

Die Gasreinigung kann durch konventionelle Wäschen erfolgen. Bevorzugt weist die erfindungsgemäße Vorrichtung jedoch getrennte Aufarbeitungsstufen für die Schwefelverunreinigungen und das CO₂ auf. So kann die erfindungsgemäße Vorrichtung eine 2-stufige Schwefelreinigung aufweisen, wobei in der ersten Stufe über die Erzeugung der erforderlichen SO₂-Menge elementarer Schwefel katalytisch erzeugt wird und die zweite Stufe eine Aktivkohlestufe ist, in der die Aktivkohle mit bestimmten Verbindungen wie Jod bzw. Jodverbindungen, Eisenverbindungen und Oxidationskatalysatoren dotiert ist.

CO₂ kann getrennt über eine Druck-Wechsel-Adsorbtionsanlage aufgearbeitet werden oder über eine konventionelle Wäsche. Beide Anlagen können in der erfindungsgemäßen Vorrichtung installiert sein.

Die erfindungsgemäße Vorrichtung weist eine Methanol-Anlage auf, die aus mehreren Methanol-Reaktoren besteht, vorzugsweise 3 - 5 Reaktorstufen, besonders bevorzugt 4 Reaktorstufen mit Zwischenabzügen. Die Zwischenabzüge zum Abziehen des Methanols aus dem Reaktionsgleichgewicht können unterschiedlich ausgebildet sein, bevorzugt ist jedoch die Ausbildung als Extraktionsstufe. Die Extraktionsstufe ist jeweils hinter eine Methanol-Reaktorstufe geschaltet.

Die Erfindung soll mit Hilfe der Figuren näher erläutert werden. Die Figuren sind beispielhaft, jedoch nicht als limitierend anzusehen, da zahlreiche abgewandelte Anordnungen erfindungsgemäß möglich sind.

Figur 1 stellt beispielhaft eine erfindungsgemäße Hochtemperatur-Konvertierung dar.

Figur 2 stellt beispielhaft eine erfindungsgemäße Gasreinigung dar.

Figur 3 stellt beispielhaft eine erfindungsgemäße Gesamtanlage dar.

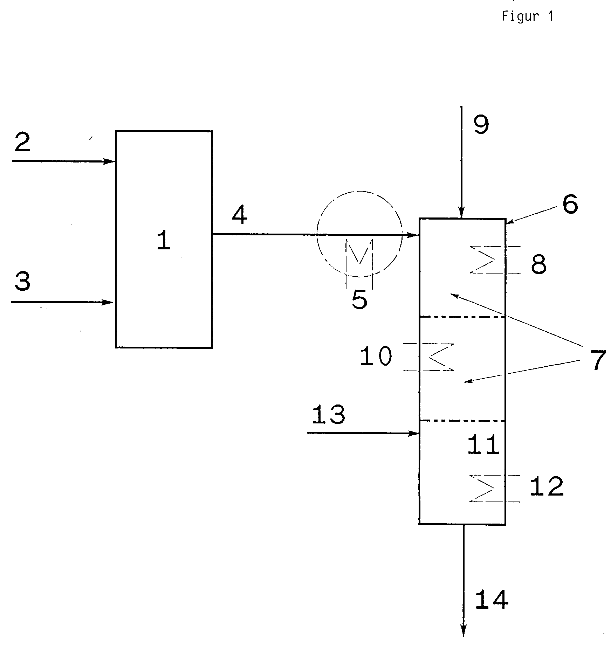

In Figur 1 stellt 1 den Vergaser dar, in den über 2 und 3 Wasser/Dampf bzw. zu vergasendes Kohlenstoff enthaltendes Material zugeführt werden. Über 4 gelangt das Syntheserohgas in den Konvertierungsreaktor 6, der aus einer Konvertierungszone 7 und einer Kühlzone 11 besteht. In Zuführung 4 kann ein indirekter Wärmetauscher 5 angeordnet sein. In 7 kann über 9 Quenchwasser bzw. Dampf eingeleitet werden. In 7 können indirekte Wärmetauscher 8 und 10 angeordnet sein. 11 ist die Abkühlzone des Konvertierungsreaktors, in die über 13 Wasser bzw. Niederdruckdampf zugeführt werden kann, wobei das Wasser vorzugsweise eingedüst wird. Auch 11 kann mit indirekter Kühlung 12 ausgestattet sein. Über 14 wird das abgekühlte Synthesegasgemisch aus dem Konvertierungsreaktor entnommen.

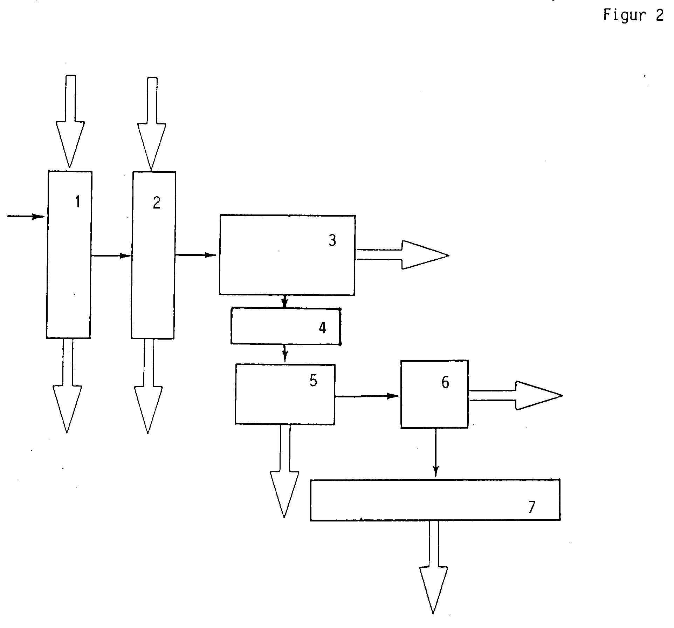

In Figur 2 können in 1 und 2 bei Einsatz Halogen und Stickstoff enthaltender Materialien, insbesondere Abfallmaterialien, Halogenwasserstoffsäuren, Ammoniak und ggf. Reststaub ausgewaschen werden. In 3 erfolgt die katalytische Entschwefelung. In 4 können COS, CS₂ und ggf. HCN hydrolysiert werden. Eine solche Hydrolysevorrichtung kann auch 1 vorgeschaltet werden. In 5 erfolgt die Umsetzung von Restschwefelwasserstoff über dotierter Aktivkohle. In 6 kann CO₂ durch Druckwechseladsorption entfernt werden. Das gereinigte Gas gelangt nach 7 zur Methanolsynthese.

Wie bereits aufgeführt, kann anstelle der geschilderten Anlage auch eine konventionelle Gaswäsche mit separater Rückgewinnung der Gasgemischkomponenten eingesetzt werden.

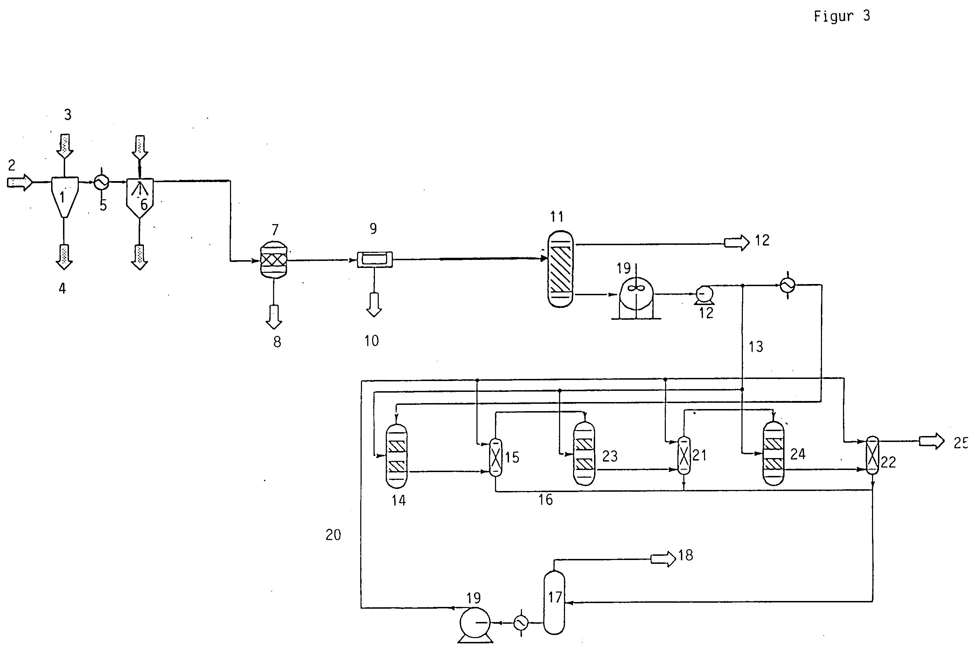

In Figur 3 stellt 1 eine Vergasungsvorrichtung dar, in die über 2 Kohlenstoff enthaltende Materialien zugeführt werden. Über 3 gelangen Sauerstoff ggf. Dampf in Reaktor 1. Bei 4 wird Schlacke abgezogen.

In 6 erfolgt die Hochtemperatur-Konvertierung, ggf. über Wärmetauscher 5.

Aus 6 strömt das Synthesegas in Reaktor 7, in dem Schwefelwasserstoff zu Schwefel oxidiert wird. Bei 8 wird flüssiger Schwefel abgezogen. Eine Nachreinigung zur Entfernung kleiner Restmengen von Schwefelwasserstoff erfolgt in Aktivkohlebett 9. Zu regenerierende Aktivkohle wird bei 10 abgezogen. Das Synthesegas fließt in den Druckwechseladsorber 11, der im allgemeinen mehrstufig ist. Bei 12 wird CO₂ abgezogen. In Mischtank 19 kann, falls erforderlich, Nachmischen zum Erreichen des benötigten CO-H₂-Verhältnisses erfolgen. Wie oben bereits erwähnt, kann erfindungsgemäß auch ggf. auf die Konvertierung und ggf. auch auf die Druckwechse ladsorption und Rückmischung verzichtet werden. Mittels Kompressor 12 wird das Synthesegas nun in den ersten Methanolreaktor gepumpt. Über Leitung 13 kann im Falle eines Abschnittsreaktors Kaltgas zugeführt werden. Aus Reaktor 14 fließt das Produktgemisch in die erste Extraktionsstufe 15. Der flüssige Methanolextrakt fließt über 16 in Desorptionsstufe 17. Dort wird nach Entspannen bei 18 Methanol dampfförmig abgezogen. Das Extraktionsmittel wird mittels Pumpe 19 und Leitung 20 in die Extraktionsstufen 15, 21 und 22 zurückgepumpt. Das Synthesegas strömt aus 15 bzw. 21 in die Methanolsynthesereaktoren 23 und 24. Aus Extraktionsstufe 22 wird nicht umgesetztes Gas über 25 abgezogen, das wie bereits oben erläutert, in die Vergasungsreaktoren eingeleitet werden kann. Wie bereits oben ausgeführt, kann die Methanolsynthese-Anlage auch aus weniger oder mehr Stufen bestehen, wie z. B. 1, 2, 3, 4, 5 oder mehr Stufen.

## Patentansprüche

1. Verfahren zur Erzeugung von Methanol, dadurch gekennzeichnet, daß in einem Vergaser erzeugtes Synthesegas einer Hochtemperaturkonvertierung zugeführt wird und daß aus dem konvertierten Gas nach Gasreinigung in einer Methanolanlage mit Methanolzwischenabzug Methanol erzeugt wird.

2. Verfahren nach Anspruch 1, dadurch gekennzeichnet, daß als Schlackenbadvergaser der Thermoselect-Vergaser eingesetzt wird.

3. Verfahren nach Anspruch 1, dadurch gekennzeichnet, daß als Vergaser ein Zyklonvergaser eingesetzt wird.

4. Verfahren nach wenigstens einem der Ansprüche 1 bis 3, dadurch gekennzeichnet, daß Kohlenstoff enthaltende Abfälle allein oder im Gemisch mit sonstigen Kohlenstoff enthaltenden Materialien vergast werden.

5. Verfahren nach wenigstens einem der Ansprüche 1 bis 4, dadurch gekennzeichnet, daß rohes Synthesegas in die Konvertierung eingesetzt wird.

6. Verfahren nach wenigstens einem der Ansprüche 1 bis 5, dadurch gekennzeichnet, daß die Konvertierung in Abwesenheit von Katalysatoren durchgeführt wird.

7. Verfahren nach wenigstens einem der Ansprüche 1 bis 6, dadurch gekennzeichnet, daß die Konvertierung in einem Temperaturbereich von 600 bis 1.200 °C, bevorzugt von 600 bis 1.000°C und besonders bevorzugt von 700 bis 900 °C durchgeführt wird.

8. Verfahren nach wenigstens einem der Ansprüche 1 bis 7, dadurch gekennzeichnet, daß das Gasgemisch nach Konvertierung auf eine Temperatur von 50 bis 200 °C, bevorzugt von 50 bis 150 °C und besonders bevorzugt von 70 bis 130 °C abgeschreckt wird.
